# EUROPEAN PATENT SPECIFICATION

(11) **EP 3 435 877 B1**
(45) Date of publication and mention of the grant of the patent: **30.06.2021**
(21) Application number: 17715093.5
(22) Date of filing: 30.03.2017
(51) Int. Cl.: A61B 8/08, A61B 8/12, A61B 8/00, A61B 5/00

(54) **INTRAVASCULAR DEVICES, SYSTEMS, AND METHODS UTILIZING PHOTOACOUSTIC, ULTRASOUND, AND OPTICAL COHERENCE TOMOGRAPHY IMAGING TECHNIQUES**
INTRAVASKULÄRE VORRICHTUNGEN, SYSTEME UND VERFAHREN UNTER VERWENDUNG VON PHOTO-AKUSTISCHEN, ULTRASCHALL- UND OPTISCHEN KOHÄRENZTOMOGRAPHIEBILDGEBUNGSTECHNIKEN
DISPOSITIFS, SYSTÈMES ET PROCÉDÉS INTRAVASCULAIRES UTILISANT DES TECHNIQUES D'IMAGERIE PHOTOACOUSTIQUES, ÉCHOGRAPHIQUES ET DE TOMOGRAPHIE PAR COHÉRENCE OPTIQUE

(30) Priority: 30.03.2016 US 201662315275 P
(43) Date of publication of application: 06.02.2019
(73) Proprietor: Koninklijke Philips N.V., 5656 AG Eindhoven (NL)
(72) Inventor: STIGALL, Jeremy, 5656 AE Eindhoven (NL); SAROHA, Princeton, 5656 AE Eindhoven (NL)
(74) Representative: de Haan, Poul Erik
(86) International application number: PCT/EP2017/057479
(87) International publication number: WO 2017/167842

(56) References cited:
- WO-A1-2015/054243
- CN-A- 104 257 342
- US-A1- 2011 098 572

## Description

### TECHNICAL FIELD

The present disclosure relates generally to imaging and mapping vascular pathways and surrounding tissue with photoacoustic and ultrasound modalities.

### BACKGROUND

Innovations in diagnosing and verifying the level of success of treatment of disease have migrated from external imaging processes to internal diagnostic processes. In particular, diagnostic equipment and processes have been developed for diagnosing vasculature blockages and other vasculature disease by means of ultra-miniature sensors placed upon the distal end of a flexible measurement apparatus such as a catheter, or a guide wire used for catheterization procedures. For example, known medical sensing techniques include angiography, intravascular ultrasound (IVUS), forward looking IVUS (FL-IVUS), fractional flow reserve (FFR) determination, a coronary flow reserve (CFR) determination, optical coherence tomography (OCT), trans-esophageal echocardiography, and image-guided therapy.

For example, intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. There are two general types of IVUS devices in use today: rotational and solid-state (also known as synthetic aperture phased array). For a typical rotational IVUS device, a single ultrasound transducer element is located at the tip of a flexible driveshaft that spins inside a plastic sheath inserted into the vessel of interest. In side-looking rotational devices, the transducer element is oriented such that the ultrasound beam propagates generally perpendicular to the longitudinal axis of the device. In forward-looking rotational devices, the transducer element is pitched towards the distal tip so that the ultrasound beam propagates more towards the tip (in some devices, being emitted parallel to the longitudinal centerline). The fluid-filled sheath protects the vessel tissue from the spinning transducer and driveshaft while permitting ultrasound signals to propagate from the transducer into the tissue and back. As the driveshaft rotates, the transducer is periodically excited with a high voltage pulse to emit a short burst of ultrasound. The same transducer then listens for the returning echoes reflected from various tissue structures. The IVUS medical sensing system may assemble a two dimensional display of the tissue, vessel, heart structure, etc. from a sequence of pulse/acquisition cycles occurring during a single revolution of the transducer. In order to image a length of a vessel, the transducer element may be drawn through the vessel as it spins.

In contrast, solid-state IVUS devices utilize a scanner assembly that includes an array of ultrasound transducers connected to a set of transducer controllers. In side-looking and some forward-looking IVUS devices, the transducers are distributed around the circumference of the device. In other forward-looking IVUS devices, the transducers are a linear array arranged at the distal tip and pitched so that the ultrasound beam propagates closer to parallel with the longitudinal centerline. The transducer controllers select transducer sets for transmitting an ultrasound pulse and for receiving the echo signal. By stepping through a sequence of transmit-receive sets, the solid-state IVUS system can synthesize the effect of a mechanically scanned transducer element but without moving parts. Since there is no rotating mechanical element, the sensor array can be placed in direct contact with the blood and vessel tissue with minimal risk of vessel trauma. Furthermore, because there is no rotating element, the interface is simplified. The solid-state scanner can be wired directly to the medical sensing system with a simple electrical cable and a standard detachable electrical connector. While the transducers of the scanner assembly do not spin, operation is similar to that of a rotational system in that, in order to image a length of a vessel, the scanner assembly is drawn through the vessel while stepping through the transmit-receive sets to produce a series of radial scans.

Rotational and solid-state state IVUS are merely some examples of imaging modalities that sample a narrow region of the environment and assemble a two- or three-dimensional image from the results. Other examples include optical coherence tomography (OCT), which has been used in conjunction with ultrasound systems. One of the key challenges using these modalities with in a vascular pathway is that they are limited in gathering data on anatomy beyond the vessel walls. Although OCT imaging may yield higher resolution than IVUS imaging, OCT has particularly limited penetration depth and may take more time to image a region of tissue.

Another recent biomedical imaging modality is photoacoustic imaging. Photoacoustic imaging devices deliver a short laser pulse into tissue and monitor the resulting acoustic output from the tissue. Due to varying optical absorption throughout the tissue, pulse energy from the laser pulse causes differential heating in the tissue. This heating and associated expansion leads to the creation of sound waves corresponding to the optical absorption of the tissue. These sound waves can be detected and an image of the tissue can be generated through analysis of the sound waves and associated vascular structures can be identified, as described in U.S. Patent Publication 2013/0046167 titled "SYSTEMS AND METHODS FOR IDENTIFYING VASCULAR BORDERS".

US 2011/098572 A1 relates to an imaging probe for a biological sample that includes an OCT probe and an ultrasound probe combined with the OCT probe in an integral probe package capable of providing by a single scanning operation images from the OCT probe and ultrasound probe to simultaneously provide integrated optical coherence tomography (OCT) and ultrasound imaging of the same biological sample.

WO 2015/054243 A1 relates to a multimodality imaging system including ultrasound, optical coherence tomography (OCT), photoacoustic (PA) imaging, florescence imaging and endoscopic catheter for imaging inside the gastrointestinal tract with real-time automatic image co-registration capability, including: an ultrasound subsystem for imaging; an optical coherence tomography (OCT) subsystem for imaging, a PA microscopy or tomography subsystem for imaging and a florescence imaging subsystem for imaging.

Accordingly, for these reasons and others, the need exists for improved systems and techniques that allow for the mapping of vascular pathways and surrounding tissue.

### SUMMARY

The object of the present invention is solved by the subject-matter of the independent claim; further embodiments are incorporated in the dependent claims. Embodiments of the present disclosure provide a mapping system that combines photoacoustic and IVUS imaging system on a measurement apparatus configured to be placed in a vascular pathway. The system may allow for combinations of three mapping modalities: ultrasound, photoacoustic, and OCT. The sensor array may be rotatable around an axis of the measurement apparatus and allow the system to map vascular pathways and surrounding tissue.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig 1A is a diagrammatic schematic view of a medical sensing system according to some embodiments of the present disclosure.
Fig IB is a diagrammatic schematic view of a medical sensing system according to some embodiments of the present disclosure.
Fig 1C is a diagrammatic schematic view of a medical sensing system with an exemplary sensor array according to some embodiments of the present disclosure.
Fig ID is a diagrammatic schematic view of a medical sensing system with another exemplary sensor array according to some embodiments of the present disclosure.
Fig 2A is a diagrammatic schematic view of a medical sensing system with a sensor array according to an embodiment of the present disclosure.
Fig 2B is a diagrammatic schematic view of a medical sensing system with a sensor array according to another embodiment of the present disclosure.
Fig 2C is a diagrammatic schematic view of a medical sensing system with a sensor array according to another embodiment of the present disclosure.
Fig 2D is a diagrammatic schematic view of a medical sensing system with a sensor array according to another embodiment of the present disclosure.
Fig 2E is a diagrammatic schematic view of a medical sensing system with a sensor array according to another embodiment of the present disclosure.
Fig. 3 is a diagrammatic, perspective view of a vascular pathway and surrounding tissue with an instrument positioned within the pathway according to an embodiment of the present disclosure.
Fig. 4 is a flow diagram of a method for mapping a vascular pathway with a sensor array according to an example provided for better understanding.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices and systems, and any further application of the principles of the present disclosure are fully contemplated and included within the present disclosure as would normally occur to one skilled in the art to which the disclosure relates. For example, while the intravascular sensing system is described in terms of cardiovascular imaging, it is understood that it is not intended to be limited to this application. The system is equally well suited to any application requiring imaging within a confined cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

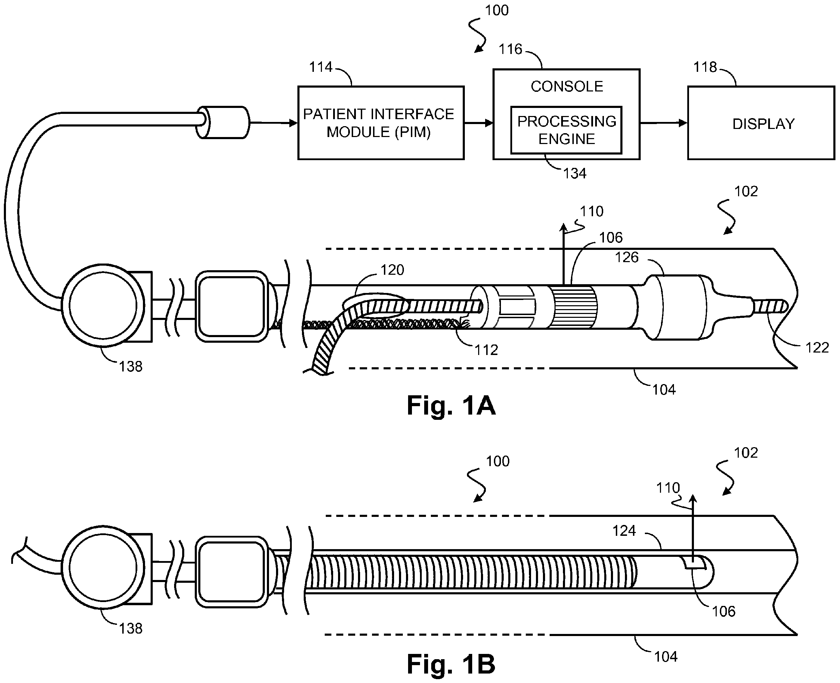

Fig. 1A is a diagrammatic schematic view of a medical sensing system 100 according to some embodiments of the present disclosure. The medical sensing system 100 includes a measurement apparatus 102 (such as a catheter, guide wire, or guide catheter). As used herein, "measurement apparatus" or "flexible measurement apparatus" includes at least any thin, long, flexible structure that can be inserted into the vasculature of a patient. While the illustrated embodiments of the "measurement apparatus" of the present disclosure have a cylindrical profile with a circular cross-sectional profile that defines an outer diameter of the flexible measurement apparatus 102, in other instances, all or a portion of the flexible measurement apparatus 102 may have other geometric cross-sectional profiles (*e.g*., oval, rectangular, square, elliptical, etc.) or non-geometric cross-sectional profiles. Flexible measurement apparatus 102 may include, for example, guide wires, catheters, and guide catheters. In that regard, a catheter may or may not include a lumen extending along all or a portion of its length for receiving and/or guiding other instruments. If the catheter includes a lumen, the lumen may be centered or offset with respect to the cross-sectional profile of the device.

The medical sensing system 100 may be utilized in a variety of applications and can be used to assess vessels and structures within a living body. To do so, the measurement apparatus 102 is advanced into a vessel 104. Vessel 104 represents fluid filled or surrounded structures, both natural and man-made, within a living body that may be imaged and can include for example, but without limitation, structures such as: organs including the liver, heart, kidneys, as well as valves within the blood or other systems of the body. In addition to imaging natural structures, the images may also include man-made structures such as, but without limitation, heart valves, stents, shunts, filters and other devices positioned within the body. The measurement apparatus 102 includes one or more sensors 106 disposed along the length of the apparatus 102 to collect diagnostic data regarding the vessel 104. In various embodiments, the one or more sensors 106 correspond to sensing modalities such as IVUS imaging, pressure, flow, OCT imaging, transesophageal echocardiography, temperature, other suitable modalities, and/or combinations thereof.

In the exemplary embodiment of Fig. 1A, the measurement apparatus 102 includes a solid-state IVUS device, and the sensors 106 include one or more IVUS ultrasound transducers and/or photoacoustic transducers and associated control. As used herein, a "photoacoustic transducer" includes at least a sensor configured to detect photoacoustic waves generated as a result of the interaction of optical pulses with tissue. In one embodiment, a photoacoustic transducer utilizes the same ultrasound detection mechanism as an IVUS ultrasound transducer. In some implementations, a single transducer can serve as both an IVUS transducer and a photoacoustic transducer. In another embodiment, a photoacoustic transducer uses a dedicated photoacoustic wave detection mechanism distinct from that of an IVUS ultrasound transducer. The system of Fig. 1A may include aspects of phased-array IVUS devices, systems, and methods associated with the Eagle Eye® Platinum catheter available from Volcano Corporation as well as those described in U.S. Patent No. 7,846,101 and/or U.S. Patent Application No. 14/812,792, filed July 29, 2015 (published as US 2016/0029999 A1).

In another embodiment, a photoacoustic transducer uses a dedicated photoacoustic wave detection mechanism distinct from that of an IVUS ultrasound transceiver. As used herein, "optical emitter" can include an optical source such a laser emitter, as well as an optical device used to transmit optical signals, such as lenses, fibers, and optical ports. As used herein, "optical transceiver" can be any device used to receive and measure optical signals, such as photo detectors and charge-coupled devices (CCD).

The sensors 106 may be arranged around the circumference of the measurement apparatus 102 and positioned to emit ultrasound energy radially 110 in order to obtain a cross-sectional representation of the vessel 104 and the surrounding anatomy. When the sensors 106 are positioned near the area to be imaged, the control circuitry selects one or more IVUS transducers to transmit an ultrasound pulse that is reflected by the vessel 104 and the surrounding structures. The control circuitry also selects one or more transducers to receive the ultrasound echo signal. By stepping through sequences of transmit-receive sets, the medical sensing system 100 system can synthesize the effect of a mechanically scanned transducer element without moving parts.

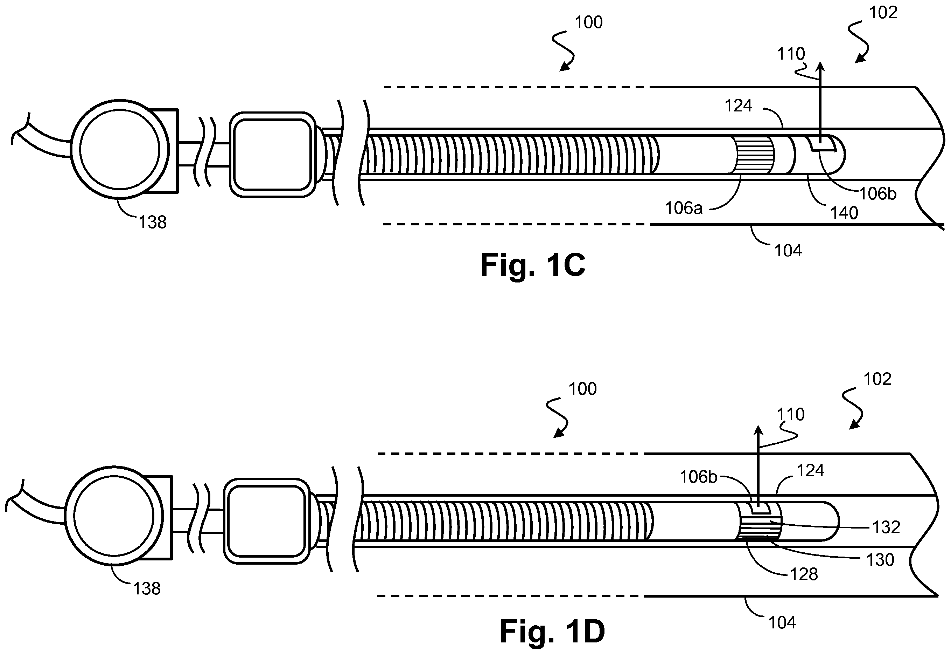

In one embodiment, the sensors 106 are disposed circumferentially around a distal portion of the measurement apparatus 102. In another embodiment, the sensors 106 are contained within the body of the measurement apparatus 102. In other embodiments, the sensors 106 are disposed radially across the measurement apparatus 102, on a movable drive member connected to the measurement apparatus 102, or on one or more planar arrays connected to the measurement apparatus 102. More examples of sensor placement are shown in Figs. 1C, ID, and 2A-2E.

In some embodiments, the processing engine 134, which may be included in the console 116, combines the imaging data acquired from both the IVUS and photoacoustic modalities into a single visualization. This use of both IVUS and photoacoustic modalities may provide a number of advantages over traditional systems using a single modality. First, the addition of photoacoustic sensors may allow for higher resolution mapping than traditional IVUS methods alone. Second, the combination of IVUS and photoacoustic modalities may allow for faster imaging speeds than OCT imaging or other methods. Third, the combination may allow for two-dimensional and/or three-dimensional imaging of the tissue surrounding vascular pathways. Fourth, the use of photoacoustic imaging may expand the diagnostic scope of an IVUS mapping procedure by including more of the surrounding tissue. In particular, the combined IVUS and photoacoustic mapping can allow for detection of certain types of cancers, tissue damage, and the mapping of multiple vascular pathways without sacrificing the dependability of ultrasound in detecting plaques, stenosis, and other forms of vascular diseases. Fifth, combining these two modalities may allow substantial costs savings because existing IVUS systems may be adapted to mapping systems using both modalities. Sixth, due to the interaction of optical pulses with tissue and the omni-directional emission of photoacoustic waves from the tissue, an optical pulse need not be emitted along the same axis as the transducer. This allows for more flexibility in carrying out combined photoacoustic and IVUS procedures, and may allow for precise mapping procedures even along deep or convoluted vascular pathways. Seventh, the mapping capabilities of the present disclosure may be integrated with some forms of laser therapy. For example, diagnosis of diseases in tissue may be accomplished using the optical emitter in diagnostic mode. After a diagnosis, the optical emitter can be switched to a treatment mode. In this regard, the map of the vasculature and surrounding tissue may be used to guide the application of the treatment. After the optical treatment is finished, the optical emitter can be switched back to diagnostic mode to confirm treatment of the diseased portion of tissue.

Sensor data may be transmitted via a cable 112 to a Patient Interface Module (PIM) 114 and to console 116, as well as to the processing engine 134 which may be disposed within the console 116. Data from the one or more sensors 106 may be received by a processing engine 134 of the console 116. In other embodiments, the processing engine 134 is physically separated from the measurement apparatus 102 but in communication with the measurement apparatus (e.g., via wireless communications). In some embodiments, the processing engine 134 is configured to control the sensors 106. Precise timing of the transmission and reception of signals may be used to map vascular pathways 104 in procedures using both IVUS and photoacoustic modalities. In particular, some procedures may involve the activation of sensors 106 to alternately transmit and receive signals. In systems using one or more IVUS transducers that are configured to receive both photoacoustic and ultrasound signals, the processing engine 134 may be configured to control the state (e.g., send/receive) of one or more transducers during the mapping of the vascular pathway and surrounding tissue.

Moreover, in some embodiments, the processing engine 134, PIM 114, and console 116 are collocated and/or part of the same system, unit, chassis, or module. Together the processing engine 134, PIM 114, and/or console 116 assemble, process, and render the sensor data for display as an image on a display 118. For example, in various embodiments, the processing engine 134, PIM 114, and/or the console 116 generates control signals to configure the sensor 106, generates signals to activate the sensor 106, performs amplification, filtering, and/or aggregating of sensor data, and formats the sensor data as an image for display. The allocation of these tasks and others can be distributed in various ways between the processing engine 134, PIM 114, and the console 116.

Sill referring to Fig. 1A, a pullback device 138 may be connected to the measurement apparatus 102. In some embodiments, the pullback device 138 is configured to pull a measurement apparatus 102 through a vascular pathway 104. The pullback device 138 may be configured to pull the measurement apparatus at one or more fixed velocities and/or fixed distances. In other instances, the pullback device 138 may be configured to pull the measurement apparatus at variable speeds and/or variable distances. The pullback device 138 may be selectively connected to the measurement apparatus 102 by mechanical connections such as male/female plug interactions, mechanical couplings, fasteners, and/or combinations thereof. Further, in some instances the pullback device 138 may be mechanically coupled and/or integrated with the PIM 114. In such instances, connection of the measurement apparatus 102 to the PIM 114 can couple the pullback device 138 to the measurement apparatus 102. The pullback device 138 may be slid across a cable, track, wire, or ribbon. In some embodiments, the pullback device 138 is in communication with one or more of a processing engine 134, a PIM 114, or a console 116. Furthermore, the pullback device 138 may be controlled by signals sent through a processing engine 134, a PIM 114, or a console 116. The pullback device 138 may also be placed in communication with another motivation device such as an actuator to drive an external optical emitter. In some embodiments, an actuator is synched with the pullback device 138 to synchronously move an external optical emitter and a measurement apparatus 102.

In addition to various sensors 106, the measurement apparatus 102 may include a guide wire exit port 120 as shown in Fig. 1A. The guide wire exit port 120 allows a guide wire 122 to be inserted towards the distal end in order to direct the member 102 through a vascular structure (*i.e*., the vessel) 104. Accordingly, in some instances the measurement apparatus 102 is a rapid-exchange catheter. Additionally or in the alternative, the measurement apparatus 102 can be advanced through the vessel 104 inside a guide catheter 124. In an embodiment, the measurement apparatus 102 includes an inflatable balloon portion 126 near the distal tip. The balloon portion 126 is open to a lumen that travels along the length of the IVUS device and ends in an inflation port (not shown). The balloon 126 may be selectively inflated and deflated via the inflation port. In other embodiments, the measurement apparatus 102 does not include balloon portion 126.

Fig. IB is a schematic view of a system that includes an alternative measurement apparatus 102 according to some embodiments of the present disclosure. The measurement apparatus 102 of Fig. IB is typical of a rotational device such as a rotational IVUS ultrasound system and the one or more sensors 106 include one or more IVUS transducers arranged to emit ultrasound energy in a radial direction 110, as well as one or more photoacoustic transducers. Again, a single transducer may serve as both an IVUS transducer and a photoacoustic transducer. In such an embodiment, the one or more sensors 106 may be mechanically rotated around a longitudinal axis of the measurement apparatus 102 to obtain a cross-sectional representation of the vessel 104. The system of Fig. IB may include aspects of rotational IVUS devices, systems, and methods associated with the Revolution® catheter available from Volcano Corporation as well as those described in U.S. Nos. 5,243,988, 5,546,948, and 8,104,479 and/or U.S. Patent Application No. 14/837,829, filed August 27, 2015 (published as US 2016/0058414 A1).

In some embodiments, sensors 106 include an OCT transceiver or an optical emitter configured to emit optical pulses from within the vascular pathway. An optical emitter may be configured to rotate around the measurement apparatus 102. Other embodiments incorporate other combinations of sensors. No particular sensor or combination of sensors is required for any particular embodiment.

Figs. 1C and 1D show further examples of a measurement apparatus 102 as contemplated by the present disclosure. In particular, the composition and placement of sensors 106 may be varied on the measurement apparatus 102. For example, Fig. 1C shows a measurement apparatus 102 which includes solid-state sensors 106a (also known as phased array sensors) and a rotational sensor 106b. In the example of Fig. 1C, the rotational sensor 106b is disposed on a drive member 140 that is attached to the measurement apparatus 102. Sensors 106 may include IVUS transducers, IVUS emitters, photoacoustic transducers, and optical emitters. The rotational sensor 106b may include an optical emitter or an ultrasound transducer. In some embodiments, the drive member 140 is attached to the measurement apparatus 102 with a drive shaft or movable hinge. The drive member 140 may be configured to rotate with respect to a longitudinal axis of the measurement apparatus 102. In some cases, the solid-state sensors 106a are attached directly to the measurement apparatus 102 and remain relatively stationary with respect to the rotating drive member 140. In some embodiments, the solid-state sensors 106a are disposed circumferentially around the measurement apparatus 102. The rotational sensor 106b may be configured to rotate around the measurement apparatus 102 in full 360° arcs. Alternatively or additionally, the rotational sensor is configured to rotate in arcs of 270°, 180°, 90°, or arcs of various other measurements. The direction of rotation of the rotational sensor 106b may vary along the length of the vascular pathway.

Fig. ID shows a measurement apparatus 102 that includes a sensor array 128. In the example if Fig. ID, the sensor array 128 may be configured to rotate with respect to a longitudinal axis of the measurement apparatus 102. In particular, the sensor array 128 may include sensors and emitters including IVUS transducers, IVUS emitters, photoacoustic transducers, and optical emitters. In some embodiments, the sensor array 128 includes sensors of at least two different types or modalities. For example, the sensor array 128 may include one or more rotational sensors 106a as well as sensors of a first type 130 and sensors of a second type 132. In the example of Fig. ID, the sensors of the first and second types 130, 132 are disposed on the array 128 in an alternating manner. In some embodiments (not shown), sensors of the first and second types 130, 132 are disposed on the array 128 in a checkerboard configuration such that individual sensors of the first type 130 are not adjacent to each other. Additionally, sensors of the first and second types 130, 132 may take up roughly equal proportions of the area of the array 128. Although they appear as square or rectangular in the example of Fig. 1C, sensors of the first and second types 130, 132 may have circular, elliptical, polygonal, or other shapes. Sensors of the first and second types 130, 132 may be spaced across the measurement apparatus 120 or they may be placed flush against each other. In some embodiments, each type of sensor may take up roughly equal proportions of the area of the array 128 relative to the other sensor types. In other embodiments, the ratio of the surface areas of two or more sensor types on the sensor array 128 is 20% and 80%, 30% and 70%, or 40% and 60%, respectively.

In the example of Fig. ID, a sensor array 128 is shown with sensors of two or more different types 130, 132 disposed in alternating rows. These rows may be disposed radially and may extend part way or completely around the measurement apparatus 102. In some embodiments, rows of sensors placed in a staggered formation such that the ends of individual rows are not co-terminus. In some embodiments, rows of sensors are placed adjacent to each other with no space in between. Alternatively, rows of sensors are spaced across the measurement apparatus 102 with space therebetween. In some cases, 2, 3, 4, or 5 rows of alternating sensors are disposed on the measurement apparatus 102. As discussed above, the array 128 may be configured to rotate around an axis of the measurement apparatus 102.

As the measurement apparatus 102 is moved along a vascular pathway 104, the rotational sensors 106b and the sensors of the first and second types 130, 132 may be operable to image and/or map different sections of the interior of the vascular pathway. In some embodiments, the measurement apparatus 102 is moved at a slow speed so that sensors on opposite sides of the sensor array 128 are able to map the entire vascular pathway 104 individually, creating a multimodal map of the vascular pathway 104.

The sensor array 128 may also be disposed on a separate instrument in contact with the measurement apparatus 102, as shown in Fig. 1C. For example, the sensor array 128 may be disposed circumferentially on a drive member 140 which is in contact with the measurement apparatus 102 and revolves about the longitudinal axis of the measurement apparatus 102.

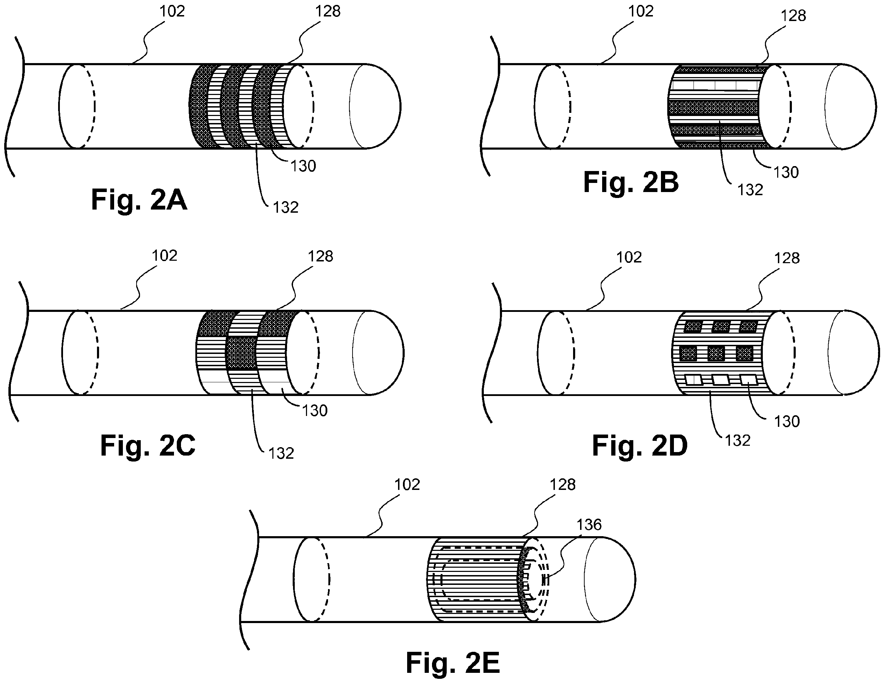

Figs. 2A-2E show examples of a sensor array 128 that may be used in conjunction with the measurement apparatus 102 according to some embodiments of the present disclosure. Only a portion of the measurement apparatus 102 is shown in Figs. 2A-2E. In some embodiments, other components are disposed distal or proximal to the sensor array 128 that are not portrayed in Figs. 2A-2E. In some embodiments, a sensor array 128 is placed in a similar position as the sensors 106 of Figs. 1A and IB. The sensor array 128 may include one or more sensors and emitters including ultrasound transducers, photoacoustic transceivers, optical emitters, and/or optical receivers. In the example of Figs. 2A-2D, the sensor array 128 is disposed around the circumference of the measurement apparatus 102, while in Fig. 2E, parts of the sensor array 128 are disposed within the body of the measurement apparatus 102. Although not shown, sensor arrays 128 may also disposed on a distal end of the measurement apparatus or on a drive member or other device separate from the measurement apparatus.

In the example of Fig. 2A, sensors of a first type 130 and sensors of a second type 132 are included in a sensor array 128. The sensors of the first and second type 130, 132 may be disposed in alternating rows. These rows may be disposed radially and may extend part way or completely around the measurement apparatus 102. In some embodiments, rows of sensors placed in a staggered formation such that the ends of individual rows are not co-terminus. In some embodiments, rows of sensors are placed adjacent to each other with no space in between. Alternatively, rows of sensors are spaced across the measurement apparatus 102 with space(s) therebetween. In some cases, 2, 3, 4, or 5 rows of alternating sensors are disposed on the measurement apparatus 102. As discussed above, the array 128 may be configured to rotate around an axis of the measurement apparatus 102.

In the example of Fig. 2B, a sensor array 128 is shown with sensors of a first type and a second type 130, 132 disposed in alternating columns. These columns of sensors may be disposed around the entire circumference of the measurement apparatus, or alternatively, may only reach around part of the circumference. In some embodiments, columns of sensors are placed adjacent to each other with no space in between. Alternatively, columns are spaced across the circumference of the measurement apparatus 102 with space therebetween.

In the example of Fig. 2C, the sensors of the first and second types 130, 132 are disposed on the array 128 in an alternating manner. In some embodiments, sensors of the first and second types 130, 132 are disposed on the array 128 in a checkerboard configuration such that individual sensors of the first type 130 are not adjacent to each other. Additionally, sensors of the first and second types 130, 132 may take up roughly equal proportions of the area of the array 128. Although they appear as square or rectangular in the example of Fig. 2C, sensors of the first and second types 130, 132 may have circular, elliptical, polygonal, or other shapes. Sensors of the first and second types 130, 132 may be spaced across the measurement apparatus 120 or they may be placed flush against each other.

In the example of Fig. 2D, a sensor array 128 is shown with sensors of the first type 130 surrounded by sensors the second type 132. In some embodiments, the ratio of the surface areas of the sensors of the first and second types 130, 132 on the sensor array 128 is 20% and 80%, 30% and 70%, or 40% and 60%, respectively. In one embodiment, sensors of the first and second types 130, 132 are disposed on the same layer and lie flush across the surface of the sensor array 128. In another embodiment, some sensors of the first and second types 130, 132 are raised relative to other sensors. For example, sensors of the first and second types 130, 132 may extend a distance of 0.25 mm, 0.5 mm, or 1 mm from the base of the sensor array 128.

In the example of Fig. 2E, a sensor array 128 is shown with concentric layers 136 of sensors. In some embodiments, layers 136 of sensors are disposed coaxially with the measurement apparatus 102. Furthermore, sensors of the first and second types 130, 132 may form alternating layers 136 in the sensor array 128. For example, a sensor layer 136 comprising ultrasound transducers may lie above a layer of photoacoustic transducers, which lies above another layer of ultrasound transducers. This arrangement may allow for a more compact measurement apparatus 102 suitable for use within a wide range of vascular passages. Other exemplary sensor arrays 128 and combinations of sensors are contemplated besides those shown in Figs. 2A-2E. For example, a sensor array 128 may combine the layers of Fig. 2E with the checkerboard layout of Fig. 2C to create a layered, alternating sensor array 128.

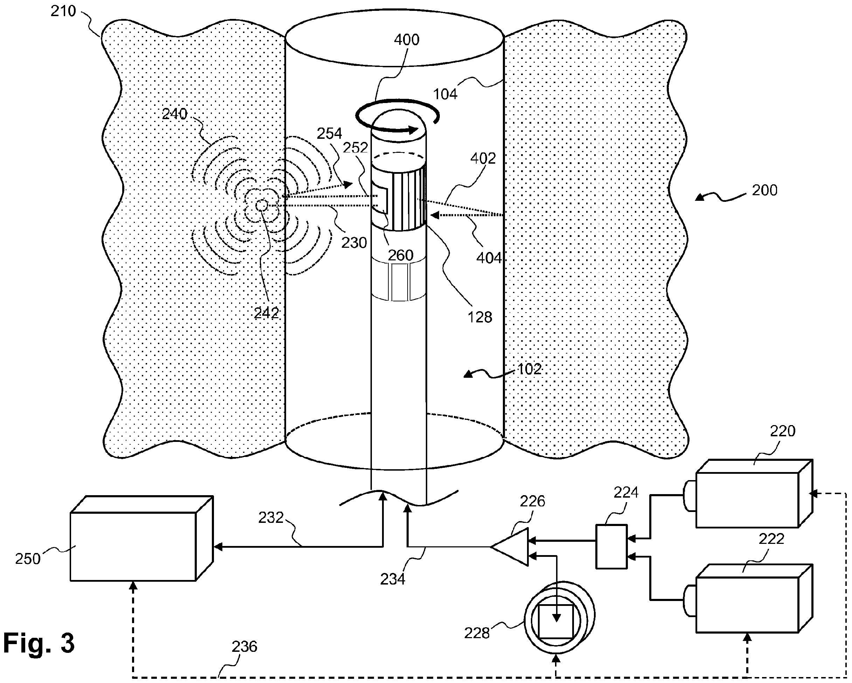

Fig. 3 is a diagrammatic, perspective view of a medical mapping system 200 as well as a vascular pathway 104 and surrounding tissue 210. In some embodiments, the medical mapping system 200 includes a measurement apparatus 102 disposed within the vascular pathway 104. The measurement apparatus 102 may be similar to the measurement apparatus 102 depicted in Figs. 1A-1D. In some embodiments, the measurement apparatus 102 is connected to and moved through the vascular pathway 104 by a pullback device 138 such as that depicted in Figs. 1A and IB. A sensor array 128 may be disposed around the measurement apparatus 102. The sensor array 128 may be any of those depicted in Figs. 2A-2E. In some embodiments, the sensor array 128 includes a plurality of ultrasound transducers which emit ultrasound signals 402 radially toward a section of the wall of the vascular pathway 104. The ultrasound signals 402 are reflected off the wall of the vascular pathway 104 and travel back toward the measurement apparatus 102 as ultrasound echo signals 404. These ultrasound echo signals 404 may be received by ultrasound transducers on the sensor array 128. In some cases, a communication system 250 controls the transducers of the sensor array 128 to emit ultrasound signals 402 and receive ultrasound echo signals 404. In some embodiments, the medical sensing system 100 is operable to map the vascular pathway 104 by mapping sections of the pathway wall as the measurement apparatus 102 is advanced through the vascular pathway 104 in direction 400.

In some embodiments, the sensor array 128 may be configured to rotate around a longitudinal axis of the measurement apparatus 102. In the example of Fig. 3, a top section of the measurement apparatus 102 with the sensor array 128 is rotated in direction 400. The speed, axis, and direction of the rotation may vary throughout a medical procedure. For example, the direction of rotation may be changed several times to allow the measurement apparatus to image an area several times or obtain additional diagnostic data on an area of interest. In some embodiments, sections of the measurement apparatus 102 are disposed within a sheath 124 such as that depicted in Fig. IB in order to protect the measurement apparatus 102 and/or vascular pathway 104. In some embodiments, some of the sensors of the sensor array 128 are rotatable around an axis of the measurement apparatus 128 while other sensors are not rotated. For example, an optical element 260, such as that shown emitting the first and second sets of optical pulses 230, 252 may be rotated while a solid-state portion of the measurement apparatus remains relatively stationary. In some cases, different layers of the measurement apparatus 128 are rotated while others remain stationary.

In some cases, an operator may move the measurement apparatus 102 through the vascular pathway 104 during the process of mapping a vascular pathway 104. In some cases, the sensor array 128 is configured to map the vascular pathway 104 independently with different modalities. For example, the vascular pathway may be mapped by an ultrasound procedure independently of procedures using photoacoustic or OCT modalities. In some embodiments, the sensor array 128 is operable to map with different combinations of modalities depending on a desired outcome of a procedure.

A first optical emitter 220 and a second optical emitter 222 are also shown in Fig. 3. In some embodiments, the first optical emitter 220 and second optical emitter 222 are laser sources that are configured to emit short laser pulses toward an area of interest within the tissue 210 of the patient. In some embodiments, the area of interest includes part of a vascular pathway 104 as well as adjacent tissue. In the example of Fig. 3, the first and second optical emitters 220, 222 are disposed externally and optical pulses from the optical emitters 220, 222 are transmitted through a route to the measurement apparatus 102. However, in other embodiments, one or more optical emitters may be placed within the vascular pathway. For example, an optical emitter or an optical element 260 may be placed directly on the measurement apparatus 102 and may form part of the sensor array 128.

The first optical emitter 220 may be configured to emit a first set of optical pulses 230 which travel on a route to the measurement apparatus 102 and ultimately are focused on a focus point 242 within the region of interest. The first set of optical pulses 230 may interact with the tissue 210 at the focus point 242, generating a series of photoacoustic waves 240 that propagate through the tissue 210 and the vascular pathway 104. The photoacoustic waves 240 may be received by sensors in the sensor array 128. In some embodiments, the sensor array 128 is disposed on the measurement apparatus 102. In other embodiments, the sensor array 128 is disposed on a separate device in contact with the measurement apparatus. The sensor array 128 may be configured to send and receive signals to map the vascular pathway.

In some embodiments, the first and second sets of optical pulses 230, 252 are routed through one or more optical fibers disposed between the measurement apparatus and the first and second optical emitters 220, 222. In some cases, optical fibers pass between the first and second optical emitters 220, 222 and a junction 224, between the junction 224 and a switch 226, between the switch 226 and a photo detector 228, and between the switch 226 and a measurement apparatus. Additionally, other optical components, including lenses, mirrors, and other reflectors form various sections of the route through which the first and second sets of optical pulses 230, 252 travel.

The second optical emitter 222 may be configured as an OCT emitter. In particular, the second optical emitter 222 may emit a second set of optical pulses 252 which travel on a route to the measurement apparatus 102 and ultimately are emitted into the region of interest. The second set of optical pulses 252 may interact with tissue 210, and a set of scattered or reflected pulses 254 may travel back toward the measurement apparatus 102. In some cases, this set of reflected pulses 254 is measured and compared to the second set of optical pulses 252, allowing for the mapping of the region of interest. In some embodiments, the second optical emitter 222 is configured with a similar functionality to the first optical emitter 220, and may emit sets of optical pulses that create photoacoustic waves that are received by the system to map a region of interest. This two-emitter approach may allow the medical mapping system 200 to achieve higher frequency of measurements, varying penetration depths, and/or improved contrast of imaging. In some embodiments, the function of the second optical emitter 222 may be changed from a photoacoustic function to an OCT function depending on the desired outcome of the procedure. For example, the medical mapping system 200 may first be used to conduct a preliminary mapping procedure with the first optical emitter 220 having a photoacoustic function and the second optical emitter 222 having an OCT function. Tissue identified by the preliminary mapping procedure as a trouble area may be re-mapped at different depths and at higher resolution with both the first optical emitter 220 and the second optical emitter 222 having a photoacoustic function. In some embodiments, the first and second optical emitters 220, 222 are combined into a single unit. The optical emitters 220, 222 may share a power source and may be operated simultaneously or independently.

In some embodiments, optical pulses emitted by the first and second optical emitters 220, 222 pass through a junction 224. This junction 224 may be adjustable and in some cases may be used to shorten or lengthen the route of the first and second sets of laser pulses 230, 252. The junction may be controllable by a communication system 250, a processing engine 134, a PIM 114, or a console 116, such as those depicted in Fig. 1. In some embodiments, the junction 224 is a motorized reflector. The junction 224 may also be included with the first and second optical emitters 220, 222 in a single housing.

The sets of optical pulses 230, 252 emitted by the first and second optical emitters 220, 222 may also pass through a switch 226. The switch 226 may include one or more optical fibers, one or more reflectors, one or more lenses, and/or other optical devices. The switch 226 may be used to route the optical pulses in different directions. For example, optical pulses traveling from the first or second optical emitters 220, 222 may be routed to a photo detector 228 by the switch. This may allow for the analysis of reflected or deflected pulses, as in OCT imaging and/or mapping procedures. In some embodiments, the switch 226 is controllable by a communication system 250, a processing engine 134, a PIM 114, or a console 116, such as those depicted in Fig. 1.

In the example of Fig. 2, the first and second optical emitters 220, 222 are in communication with a communication system 250 via connection 236. In some embodiments, the communication system 250 is the processing engine 134, the PIM 114, or the console 116 of Fig. 1A. The communication system 250 may also be connected to the measurement apparatus 102 via connection 234. Furthermore, the measurement apparatus 102 may be in direct communication with the optical emitter 220 via connection 232. In some embodiments, connections 232, 234, and 236 are cables operable to transmit electronic or optical signals. Furthermore, connection 232 may be a microcable, connection 234 may be an optical fiber, and connection 236 may be a wireless connection such as a Bluetooth or WiFi connection. Additionally, the optical emitter 220 may include a wireless signal receiver. Connection 234 may also operate to power the measurement apparatus 102 including the sensor array 128.

A communication system 250 may coordinate the operation of various elements, such as the first and second optical emitters 220, 222, junction 224, switch 226, photo detector 228, sensor array 128, and measurement apparatus 102. For example, several connections 232, 234, 236 may allow communication between the various elements. In some embodiments, the communication system 250 includes one or more of the processing engine 134, the PIM 114, or the console 116 of Fig. 1A. In particular, the communication system 250 may coordinate the operation of the first and second optical emitters 220, 222 and the sensors of the sensor array 128 by sending signals to synchronize the emission of optical pulses 230, 252 and the reception of photoacoustic signals by the sensor array 128. In some cases, the communication system 250 coordinates the operation of different sensor types on the sensor array 128. In particular, the communication system 250 may switch between ultrasound, optical, and photoacoustic functions on the sensor array 128. The operation of only one type of sensor at a time may filter out noise and yield more accurate mapping of the vascular pathway.

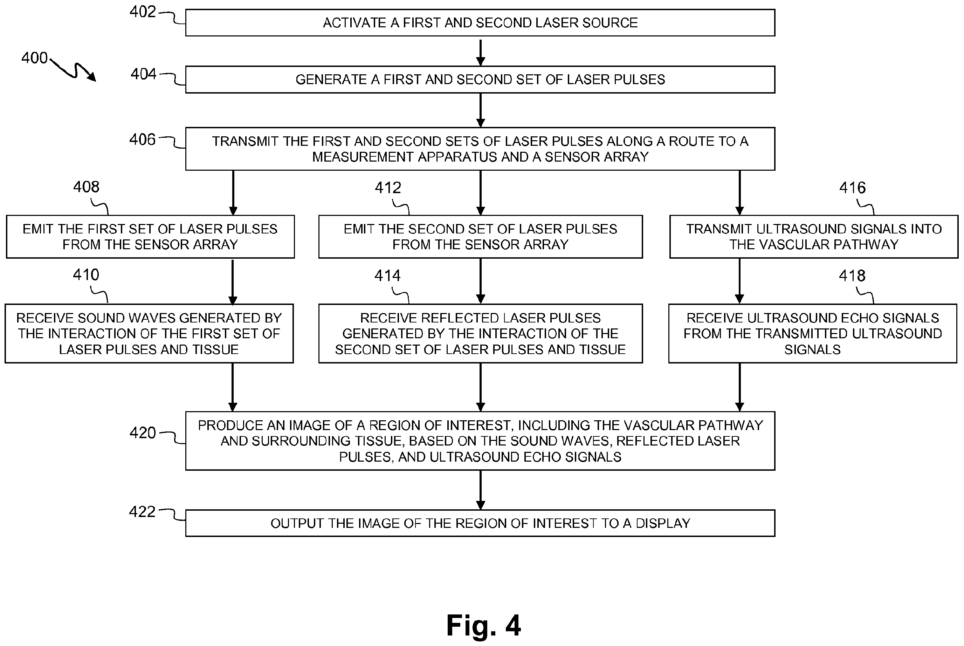

Fig. 4 is a flow chart showing a method 400 of mapping an area of interest using photoacoustic, ultrasound, and OCT modalities. It is understood that additional steps can be provided before, during, and after the steps of method 400, and that some of the steps described can be replaced or eliminated for other examples of the method. In particular, steps 408, 410, 412, 414, 416, and 418 may be performed simultaneously or in various sequences as discussed below.

At step 402, the method 400 can include activating a first and a second laser source. These laser sources may be the first and second optical emitters 220, 222 depicted in Fig. 3. In some embodiments, the first and second laser sources are disposed externally and are in communication with a measurement apparatus. In some embodiments, the measurement device is the measurement apparatus 102 depicted in Figs. 1A, 1B, 1C, 1D, 2A-2E, and 3.

The first and second laser sources may transmit laser pulses through a communication device, such as an optical fiber, to the measurement apparatus. In other embodiments, the first and second laser sources are disposed on or within the measurement apparatus. For example, the first and second laser sources may be included on a sensor array disposed on or within the measurement apparatus. In some embodiments, the sensor array is the sensor array 128 depicted in Figs. 1A, 1B, 1C, ID, and 2A-2E. The sensor array may include one or more sensors and emitters including IVUS transducers, IVUS emitters, OCT transducers, photoacoustic transceivers, and optical emitters. The two or more transducer elements may be arranged in any of the examples depicted in Figs. 1A-1D, and 2A-2E. In some cases, the first and second laser sources are activated by a communication system by means of an electronic or optical signal. This signal may be sent wirelessly, and the first and second laser sources may be equipped with a wireless signal receiver.

At step 404, the method 400 can include generating a first and second set of laser pulses. In some examples, the first and second sets of laser pulses are generated simultaneously. In other examples, the generation of the first and second sets of laser pulses occurs at different times. This may allow the laser pulses to function with different modalities without causing interference between the pulses. For example, the first set of laser pulses may be configured to produce photoacoustic waves, while the second set of laser pulses may be configured to conduct OCT imaging and/or mapping.

At step 406, the method 400 can include transmitting the first and second sets of laser pulses along a route to a measurement apparatus and a sensor array. In some embodiments, the sensor array is a solid-state array or a phased array that does not rotate as it travels through the vascular pathway 104. In other embodiments, the sensor array is a rotational array. In some embodiments, the sensor array is disposed on a revolving portion of the measurement device. In some embodiments, the sensor array is disposed circumferentially around the measurement device. The sensor array may be disposed on the measurement apparatus or alternatively, be disposed on a separate device that is connected to the measurement apparatus.

The first and second sets of laser pulses may travel across one or more optical devices to the measurement apparatus, including optical fibers, reflectors, mirrors, etc. In some cases, the first and second sets of laser pulses travel across one or more junctions and switches. This may allow an operator to control the path of the first and second sets of laser pulses.

At step 408, the method 400 can include emitting the first set of laser pulses from the sensor array. In some embodiments, the first set of laser pulses is emitted from an optical component on the sensor array, such as an optical wire or port. The first set of laser pulses may be focused on a tissue in a region of interest. In some embodiments, the region of interest includes a portion of tissue including a portion of at least one vascular pathway 104, and the measurement device may be disposed within the vascular pathway 104. The region of interest may be chosen based on a suspected or diagnosed problem in the tissue, or based on the proximity of a region of tissue to problems within a vascular pathway 104. In other embodiments, the region of interest is part of a more general mapping plan. For example, a mapping plan for a section of a vascular pathway 104 may involve the mapping of tissue surrounding the vascular pathway 104 along its length. In some embodiments, the laser pulse is emitted outwards from a measurement device that is placed within a vascular pathway. The first set of laser pulses may interact with the tissue, creating a set of sound waves that travels through the tissue and the vascular pathway 104.

At step 410, the method 400 can include receiving sound waves generated by the interaction of the first set of laser pulses and tissue. In some cases, the sensor array includes a photoacoustic sensor, which may also function with the traditional IVUS function and receive ultrasound waves. The photoacoustic sensor may be an ultrasound transducer. In other cases, the photoacoustic sensor is configured to receive only photoacoustic waves. In some embodiments, the sensor elements, including a photoacoustic sensor, are controlled by a communication system similar to communication system 250 of Fig. 3. In another embodiment, a processing engine 134 or a PIM 114 controls the operation of the sensor of the sensor array 128. Signals may be sent from processing engine 134 or the PIM 114 to sensors in the sensor array, via connector 234, causing the sensors to receive diagnostic information.

At step 412, the method 400 can include emitting the second set of laser pulses from the sensor array. In some embodiments, the second set of laser pulses may be focused on a region of tissue. A portion of the laser pulses may be scattered or reflected by interaction optical pulses with the tissue, and some of these reflected pulses may travel back toward the sensor array.

At step 414, the method 400 can include receiving the reflected laser pulses generated by the interaction of the second set of laser pulses and the tissue. In some cases, the pulses are received by one or more optical receivers on the sensor array. In particular, these optical receivers may receive the reflected laser pulses and send data on the reflected pulses to be compared with the second set of optical pulses. This may allow parts of the tissue to be imaged, for example, in an OCT modality.

At step 416, the method 400 can include transmitting ultrasound signals into the vascular pathway 104. In some embodiments, the sensor array of step 406 includes one or more ultrasound transducers which may emit ultrasound signals toward the walls of the vascular pathway 104. The transmitted ultrasound signals may be deflected off the walls of the vascular pathway 104 and propagate through the vascular pathway 104 as ultrasound echo signals.

At step 418, the method 400 can include receiving the ultrasound echo signals from the transmitted ultrasound signals. In some embodiments, an ultrasound transducer receives the ultrasound echo signals. The ultrasound transducer of steps 410, 416, and 418 may be combined in a single element. In this case, the transducer may be able to receive both sound waves and ultrasound signals. In other embodiments, the transducer elements are separate elements.

It is noted that steps 404, 406, 408, 410, 412, 414, 416, and 418 may be coordinated in various combinations and orders in the method 400. In some cases, the order of steps of method 400 may be determined based on the desired outcome of a medical procedure. For example, transmission of ultrasound signals and reception of ultrasound echo signals can occur at regular intervals throughout the method 400, while reception of photoacoustic waves and reflected laser pulses may occur sporadically. This may be the case in a medical procedure to map a vascular pathway 104 and spot-check trouble areas of tissue surrounding sections of the vascular pathway 104. Alternatively, steps 408, 410, 412, 414, 416, and 418 are performed successively. For example, each of steps 408, 410, and 416 may be performed individually before proceeding to the next step to avoid signal noise and allow for adequate signal processing. This may be the case when method 400 in used conjunction with a system where a photoacoustic sensor, optical transducer, and ultrasound transducer are each included in the sensor array. Furthermore, the steps of method 400 may be interleaved in various orders.

It is also noted that the measurement apparatus and sensor array may be moved during the operation of steps 406, 408, 410, 412, 414, 416, and 418. For example, method 400 can include rotating the sensor array about a longitudinal axis of the measurement device. In some embodiments, the sensor array is rotated throughout steps 408, 410, 412, 414, 416 and 418, as in the case where the measurement device continually maps a vascular pathway as is it pulled through the vascular pathway. In other embodiments, the sensor array is kept motionless during various steps. The rotation of the sensor array may be accomplished through the use of a drive member connected to the measurement device. In some embodiments, such as the example of Fig. 1C, parts of the sensor array are rotated around a longitudinal axis of the measurement device while other parts of the sensor array are not rotated. The rotation of the sensor array can vary in direction and rate of rotation. For example, the sensor array may be rotated 180° in a counter-clockwise direction and/or rotated in an 180° counter-clockwise direction. Rotations in each direction of 90°, 270°, 360°, and other angles are also contemplated.

At step 420, the method 400 can include producing an image of the region of interest, including the vascular pathway 104 and surrounding tissue, based on the sound waves, reflected laser pulses, and the ultrasound echo signals. In some embodiments, a processing engine 134 in communication with the sensor array produces the image of the region of interest. This image can include both two-dimensional and three-dimensional images based on the received sensor data. In some cases, the image includes a number of two-dimensional cross sections of the vascular pathway 104 and surrounding tissue.

At step 422, the method 400 can include outputting the image of the region of interest to a display. In some embodiments, this display is the display 118 depicted in Fig. 1A. The display 118 can include a computer monitor, a screen on a patient interface module (PIM) 114 or console 116, or other suitable device for receiving and displaying images.

In an examples, the method 400 repeats after step 422, such that method flow goes back to step 404 and begins again. Iteration of the method 400 may be utilized to map a vascular pathway and surrounding tissue.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A medical mapping system (100) comprising:
a first laser source (220) configured to emit a first set of laser pulses (230);
a second laser source (222) configured to emit a second set of laser pulses (252);
a measurement apparatus (102) comprising a sensor array (128) including a plurality of ultrasound transducers disposed circumferentially around the longitudinal axis of the measurement apparatus (102), adapted to emit (416) and receive (418) ultrasound signals;
a photo detector (228) in communication with the measurement apparatus (102);
wherein the measurement apparatus (102) is configured to be placed within a vascular pathway (104) in a region of interest;
wherein the measurement apparatus (102) is configured to:
transmit (408) the first set of laser pulses (230) to tissue in the region of interest;
receive (410) sound waves generated by the tissue as a result of interaction of the first set of laser pulses (230) with the tissue;
transmit (412) the second set of laser pulses (252) to tissue in the region of interest;
receive (414) a set of reflected laser pulses as a result of interaction of the second set of laser pulses (252) with the tissue;
transmit ultrasound signals (416) to tissue in the region of interest;
receive ultrasound echo signals (418) as a result of interaction of the ultrasound signals with the tissue;
wherein an optical element (260) is placed on the measurement apparatus (102) forming part of the sensor array (128), the optical element being configured to emit the first and second sets of laser pulses (230, 252);
wherein the optical element (260) is adapted to rotate around a longitudinal axis of the measurement apparatus (102) while other sensors of the sensor array (128) are adapted to remain stationary;
and wherein the medical mapping system (100) further comprises:
a processing engine (134) in communication with the measurement apparatus (102), the processing engine (134) operable to produce an image of the region of interest based on the received sound waves (410), the received reflected laser pulses (414), and the received ultrasound echo signals (418); wherein the image includes both two-dimensional and three-dimensional images based on the received sensor data; and
a display (118) in communication with the processing engine (134), the display (118) configured to visually display the image of the region of interest.

2. The medical mapping system of claim 1, further comprising a motorized reflector system configured to selectively transmit the first set of laser pulses (230) or the second set of laser pulses (252) to the measurement apparatus (102).

3. The medical mapping system of claim 1, wherein the measurement apparatus (102) includes
an optical emitter configured to transmit at least one of the first set of laser pulses or the second set of laser pulses.

4. The medical mapping system of claim 1 wherein the ultrasound transducer comprises a plurality of phased array sensors.

5. The medical mapping system of claim 3, wherein the optical emitter is disposed opposite the ultrasound transducer.

6. The medical mapping system of claim 1, wherein the ultrasound transducer is further configured to receive the sound waves generated by the tissue as a result of interaction of the optical pulses with the tissue.

## Patentansprüche

1. Ein medizinisches Darstellungssystem (100), das Folgendes umfasst:
eine erste Laserquelle (220), die einen ersten Satz von Laserimpulsen (230) emittiert;
eine zweite Laserquelle (222), die einen zweiten Satz von Laserimpulsen (252) emittiert;
ein Messgerät (102), das ein Sensorarray (128) mit mehreren Ultraschallwandlern umfasst, das rund um die Längsachse des Messgeräts (102) angeordnet ist, und das Ultraschallsignale sendet (416) und empfängt (418); einen mit dem Messgerät (102) verbundenen Fotodetektor (228);
wobei das Messgerät (102) in einem Gefäßweg (104) der zu untersuchenden Region platziert wird;
wobei das Messgerät (102) folgende Schritte durchführt:
Übertragen (408) des ersten Satzes von Laserimpulsen (230) an das Gewebe in der zu untersuchenden Region;
Empfangen (410) von Schallwellen, die vom Gewebe aufgrund der Wechselwirkung des ersten Satzes von Laserimpulsen (230) mit dem Gewebe erzeugt werden;
Übertragen (412) des zweiten Satzes von Laserimpulsen (252) an das Gewebe in der zu untersuchenden Region;
Empfangen (414) eines Satzes von reflektierten Laserimpulsen aufgrund der Wechselwirkung des zweiten Satzes von Laserimpulsen (252) mit dem Gewebe;
Senden von Ultraschallsignalen (416) an das Gewebe in der zu untersuchenden Region;
Empfangen von Ultraschallechosignalen (418) aufgrund der Wechselwirkung der Ultraschallsignale mit dem Gewebe;
wobei sich auf dem Messgerät (102) ein optisches Element (260) befindet, das einen Teil des Sensorarrays (128) bildet, wobei das optische Element den ersten und zweiten Satz von Laserimpulsen (230, 252) emittiert;
wobei sich das optische Element (260) um die Längsachse des Geräts (102) dreht, während andere Sensoren des Sensorarrays (128) stationär verbleiben; und
wobei das medizinische Darstellungssystem (100) zudem Folgendes umfasst:
einen mit dem Messgerät (102) kommunizierenden Prozessor (134), wobei der Prozessor (134) beruhend auf den empfangenen Schallwellen (410), den empfangenen reflektierten Laserimpulsen (414) und den empfangenen Ultraschallechosignalen (418) ein Bild der zu untersuchenden Region erzeugt; wobei das Bild beruhend auf den empfangenen Sensordaten sowohl zweidimensionale als auch dreidimensionale Bilder umfasst; und
eine mit dem Prozessor (134) kommunizierende Anzeige (118), wobei die Anzeige (118) das Bild der zu untersuchenden Region visuell darstellt.

2. Das medizinische Darstellungssystem gemäß Anspruch 1, das zudem ein motorisiertes Reflektorsystem umfasst, das den ersten Satz von Laserimpulsen (230) oder den zweiten Satz von Laserimpulsen (252) selektiv an das Messgerät (102) überträgt.

3. Das medizinische Darstellungssystem gemäß Anspruch 1, wobei das Messgerät (102) einen optischen Sender umfasst,
der mindestens den ersten Satz von Laserimpulsen oder den zweiten Satz von Laserimpulsen überträgt.

4. Das medizinische Darstellungssystem gemäß Anspruch 1, wobei der Ultraschallwandler mehrere Phased-Array-Sensoren umfasst.

5. Das medizinische Darstellungssystem gemäß Anspruch 3, wobei der optische Sender gegenüber dem Ultraschallwandler angeordnet ist.

6. Das medizinische Darstellungssystem gemäß Anspruch 1, wobei der Ultraschallwandler zudem die Schallwellen empfängt, die vom Gewebe aufgrund der Wechselwirkung der optischen Impulse mit dem Gewebe erzeugt werden.

## Revendications

1. Système de cartographie médicale (100) comprenant :
une première source laser (220) conçue pour émettre un premier ensemble d'impulsions laser (230) ;
une seconde source laser (222) conçue pour émettre un second ensemble d'impulsions laser (252) ;
un appareil de mesure (102) comprenant un réseau de capteurs (128) comprenant une pluralité de transducteurs ultrasonores disposés de manière circonférentielle autour de l'axe longitudinal de l'appareil de mesure (102), conçus pour émettre (416) et pour recevoir (418) des signaux ultrasonores ; un photodétecteur (228) en communication avec l'appareil de mesure (102) ;
dans lequel l'appareil de mesure (102) est conçu pour être placé dans une voie vasculaire (104) dans une zone d'intérêt ;
dans lequel l'appareil de mesure (102) est conçu :
pour transmettre (408) le premier ensemble d'impulsions laser (230) au tissu dans la zone d'intérêt ;
pour recevoir (410) des ondes sonores générées par le tissu à la suite de l'interaction du premier ensemble d'impulsions laser (230) avec le tissu ;
pour transmettre (412) le second ensemble d'impulsions laser (252) au tissu dans la zone d'intérêt ;
pour recevoir (414) un ensemble d'impulsions laser réfléchies suite à l'interaction du second ensemble d'impulsions laser (252) avec le tissu ;
pour transmettre des signaux ultrasonores (416) au tissu dans la zone d'intérêt ;
pour recevoir des signaux d'écho ultrasonore (418) à la suite de l'interaction des signaux ultrasonores avec le tissu ;
dans lequel un élément optique (260) est placé sur l'appareil de mesure (102) faisant partie du réseau de capteurs (128), l'élément optique étant conçu pour émettre les premier et second ensembles d'impulsions laser (230, 252) ;
dans lequel l'élément optique (260) est conçu pour tourner autour d'un axe longitudinal de l'appareil de mesure (102), d'autres capteurs du réseau de capteurs (128) étant conçus pour rester stationnaires ;
et dans lequel ledit système de cartographie médicale (100) comprend en outre :
un moteur de traitement (134) en communication avec l'appareil de mesure (102), le moteur de traitement (134) étant utilisable pour produire une image de la zone d'intérêt en fonction des ondes sonores (410) reçues, des impulsions laser réfléchies (414) reçues et des signaux d'écho ultrasonore (418) reçues ; dans lequel l'image comprend à la fois des images bidimensionnelles et tridimensionnelles basées sur les données de capteur reçues ; et
un affichage (118) en communication avec le moteur de traitement (134), l'affichage (118) étant conçu pour afficher visuellement l'image de la zone d'intérêt.

2. Système de cartographie médicale selon la revendication 1, comprenant en outre un système de réflecteur motorisé conçu pour transmettre sélectivement le premier ensemble d'impulsions laser (230) ou le second ensemble d'impulsions laser (252) à l'appareil de mesure (102).

3. Système de cartographie médicale selon la revendication 1, dans lequel l'appareil de mesure (102) comprend
un émetteur optique conçu pour transmettre le premier ensemble d'impulsions laser et/ou le second ensemble d'impulsions laser.

4. Système de cartographie médicale selon la revendication 1, dans lequel le transducteur ultrasonore comprend une pluralité de capteurs à réseau à commande de phase.

5. Système de cartographie médicale selon la revendication 3, dans lequel l'émetteur optique est disposé à l'opposé du transducteur ultrasonore.

6. Système de cartographie médicale selon la revendication 1, dans lequel le transducteur ultrasonore est en outre conçu pour recevoir les ondes sonores générées par le tissu à la suite de l'interaction des impulsions optiques avec le tissu.
